# FASCICULE DE BREVET EUROPEEN

(11) **EP 0 523 789 B1**
(45) Date de publication et mention de la délivrance du brevet: **04.12.1996**
(21) Numéro de dépôt: 92202093.8
(22) Date de dépôt: 09.07.1992
(51) Int. Cl.: A61L 15/26, A61L 15/44, C08K 5/10, C08L 67/04, C08J 5/18, A61L 15/64

(54) **Film souple, élastique et biodégradable en polymère à base d'acide lactique pouvant notamment convenir pour la réalisation de pansements médicaux**
Weicher elastischer und bioabsorbierbarer Film aus Milchsäurepolymer, insbesondere verwendbar zur Herstellung von medizinischen Verbänden
Soft elastic and bioresorbable film from lactic and polymer particularly useful for making medical dressings

(30) Priorité: 19.07.1991 BE 9100686
(43) Date de publication de la demande: 20.01.1993
(73) Titulaire: SOLVAY (Société Anonyme), B-1050 Bruxelles (BE)
(72) Inventeur: Grandjean, Dominique, B-1120 Bruxelles (BE)
(74) Mandataire: Dufrasne, Eugène

(56) Documents cités:
- US-A- 4 306 552

## Description

La présente invention concerne un film souple élastique et biodégradable en polymère à base d'acide lactique pouvant notamment convenir pour la réalisation de pansements médicaux.

Dans le brevet BE-A-778 126, on décrit la production d'articles chirurgicaux absorbables et notamment de pansements médicaux à partir de polymères de l'acide lactique.

Selon ce document, les polymères d'acide lactique peuvent effectivement être filés et les filaments ainsi produits peuvent être tordus, entrelacés, tressés ou être disposés parallèlement pour obtenir des tissus exploitables notamment pour la réalisation de pansements médicaux.

La réalisation de pansements selon ce document implique, dès lors, au moins des opérations successives de filage, de tissage et de conditionnement.

La demanderesse a, dès lors, envisagé de simplifier ce procédé en tentant de produire directement, par extrusion, un film mince de ces polymères qui puisse notamment convenir pour la réalisation de pansements mais elle a constaté qu'un tel film est difficilement extrudable et qu'en outre le film ainsi produit présente une élasticité et notamment un allongement à la rupture qui sont trop faibles pour pouvoir envisager son exploitation sous la forme de pansements médicaux.

Pour remédier à ces problèmes, la demanderesse a alors envisagé d'incorporer divers plastifiants dans ces polymères pour faciliter l'extrusion et tenter d'améliorer les propriétés des films obtenus mais elle a ainsi constaté que les plastifiants usuels n'apportent aucun résultat satisfaisant.

Des essais entrepris avec d'autres plastifiants notamment du type ester de glycérol se sont également révélés négatifs. Ainsi, il est apparu, en particulier, que le trioléate de glycérol et le tridécanoate de glycérol ne sont pas compatibles avec les polymères dérivés de l'acide lactique, que le propylène glycol, le trihexanoate de glycérol et le glycérol conduisent à des produits présentant une résistance à l'allongement trop faible, que le triacétate de glycérol ou triacétine donne des produits présentant une résistance thermique défectueuse et que d'autres produits enfin, tels des lactates ou des citrates, n'exercent aucun effet plastifiant.

Après de nombreux essais décevants, la demanderesse a finalement pu détecter un type de plastifiant particulier qui donne entière satisfaction tant sur le plan de l'extrusion que sur le plan des propriétés mécaniques des films obtenus.

La présente invention concerne, dès lors, un film souple, élastique et biodégradable en polymère à base d'acide lactique pouvant notamment convenir pour la réalisation de pansements médicaux qui se caractérise en ce qu'il est produit par extrusion du polymère à base d'acide lactique préalablement plastifié par incorporation de 5 à 30 % en poids du polymère d'un ester de glycérol obtenu à partir d'un acide contenant 4 atomes de carbone dans sa molécule.

Il a en effet été constaté que ce type de plastifiant permet la production directe par extrusion de films souples, de comportement élastique satisfaisant et présentant un allongement à la rupture qui est supérieur à 300 %.

Les polymères à base d'acide lactique utilisables pour la réalisation du film selon l'invention sont choisis parmi les polymères thermoplastiques résultant de l'homopolymérisation de l'acide-L-lactique, de la copolymérisation de l'acide-L-lactique et d'acide-D-lactique à raison de 99 à 55 % d'acide -L-lactique et/ou encore avec d'autres monomères copolymérisables. Ces autres monomères copolymérisables peuvent être, par exemple, l'acide glycolique, la bêta-propiolactide, la tétraméthylglycolide, la bêta-butyrolactone, la gamma-butyrolactone, la pivalolactone, l'acide alpha-hydroxyacétique, l'acide alpha-hydroxybutyrique, l'acide alpha-hydroxyisobutyrique, l'acide alpha-hydroxyvalérique, l'acide alpha-hydroxyisovalérique, l'acide alpha-hydroxycaproïque, l'acide alpha-hydroxyisocaproïque, l'acide alpha-hydroxy-alpha-éthylbutyrique, l'acide alpha-hydroxyheptanoïque, l'acide alpha-hydroxystéarique, etc.

Les copolymères utilisables peuvent être aussi bien des copolymères statistiques que des copolymères à blocs.

Les polymères préférés pour la réalisation du film selon l'invention sont les copolymères d'acide-DL-lactique et d'acide glycolique contenant de 25 à 75 % et, de préférence environ 50 %, en poids d'unités dérivées d'acide lactique. Le recours à un copolymère contenant, à la fois, des unités dérivées de l'acide-L-lactique et de l'acide-D-lactique présente l'avantage de permettre un réglage de la durée de biodégradation du film produit en agissant sur la teneur en unités dérivées de l'acide-D-lactique du copolymère, cette teneur pouvant généralement varier entre 1 et 50 %.

En règle générale, on préfère mettre en oeuvre des polymères à base d'acide lactique présentant une viscosité inhérente comprise entre 0,5 et 1.

Le recours à un plastifiant constitué par un ester de glycérol dérivé d'un acide contenant 4 atomes de carbone et, plus particulièrement, à un triester tel que le tributanoate de glycérol ou tributyrine est critique pour obtenir directement par extrusion un film souple présentant un allongement à la rupture et un comportement élastique satisfaisant, en particulier, pour l'utilisation de ce film sous la forme de pansements médicaux. Le recours à d'autres esters de glycérol dérivés d'acides contenant 4 atomes de carbone dans leur molécule tels que notamment le tri-isobutanoate de glycérol n'est toutefois pas exclu du cadre de la présente invention.

Le polymère mis en oeuvre peut avantageusement contenir de 5 à 30 % en poids de plastifiant par rapport au poids de polymère, une teneur comprise entre 20 et 25 % en poids étant toutefois préférée.

Le film extrudé peut présenter une épaisseur pouvant varier entre 25 et 1000 microns et plus, une épaisseur comprise entre 25 et 100 microns étant préférée lorsque le film est destiné à être exploité pour la production de pansements médicaux.

Le film, conforme à l'invention, présente une perméabilité à l'eau relativement faible. Toutefois, il a été constaté qu'il est possible, si on le souhaite, de produire un film présentant une perméabilité à l'eau plus élevée en incorporant dans le polymère de départ de 1 à 30 % et, de préférence, de 5 % à 25 % en poids du polymère d'au moins une cellulose alkylée. A titre d'exemple non limitatif de celluloses alkylées pouvant être utilement exploitées, on peut notamment citer l'hexapropylméthylcellulose (HPMC) et l'hexapropylcellulose (HPC) qui sont préférées.

Il a encore été constaté que le film conforme à l'invention présente une bonne perméabilité aux gaz, en particulier vis-à-vis de l'oxygène et à l'anhydride carbonique. Par ailleurs, la perméabilité à l'oxygène est encore améliorée, particulièrement en atmosphère humide, lorsque le film est extrudé au départ de compositions contenant une cellulose alkylée.

Le film conforme à l'invention peut encore, lorsqu'il est destiné à être exploité en tant que pansement médical, contenir un produit pharmaceutique quelconque tel que, par exemple, un agent anti-infectieux qui est libéré progressivement lors de la biodégradation du film.

Pour réaliser le film conforme à l'invention, il suffit, en principe, de mélanger les divers constituants puis d'extruder le mélange à l'aide d'une installation classique d'extrusion convenant pour la production de films.

Le mélange des constituants est, de préférence, réalisé dans un mélangeur, par exemple du type Hobart. Lorsque la composition à extruder doit contenir une cellulose alkylée, on préfère généralement introduire d'abord le polymère à base d'acide lactique et le plastifiant dans le mélangeur et, après une première opération de mélange, puis incorporer ensuite la cellulose alkylée.

Le mélange ainsi obtenu peut être ensuite granulé sur une extrudeuse, de préférence du type monovis et être éventuellement stocké. Dans ce dernier cas, il convient toutefois que les granules soient conservés à une température inférieure à 20°C pour éviter tout risque de prise en masse.

L'installation de production du film peut être quelconque mais il faut prendre soin d'éviter que le produit traité soit porté, durant sa mise en oeuvre, à une température pouvant entraîner la dégradation du polymère à base d'acide lactique.

Le film conforme à l'invention et son mode de réalisation sont, par ailleurs, explicités plus en détail dans les exemples illustratifs qui suivent.

### Exemple 1

Dans un mélangeur HOBART, on introduit 100 parties en poids d'un copolymère d'acide-DL-lactique et d'acide glycolique contenant 50 % en poids d'unités dérivées d'acide lactique et 22 parties en poids de tributanoate de glycérol et, après 5 minutes de mélange, 5 parties en poids d'hexapropylméthylcellulose. Après une nouvelle période de mélange de 5 minutes, on introduit le produit obtenu dans une extrudeuse monovis Reifenhauser équipée d'une granulatrice.

La vis utilisée dans cette extrudeuse exerce un taux de compression de 2,5 et les températures allant de la zone d'alimentation à la tête d'extrusion s'élèvent à 40°C - 55°C - 60°C - 65°C - 70°C.

Les granules ainsi produits sont ensuite introduits dans une extrudeuse CLEXTRAL BC 21 équipée d'une filière plate de type Verbruggen d'une largeur de 100 mm.

Les vis présentent un rapport L/D de 36 et une longueur de 900 mm. Le diamètre interne des vis est de 17 mm et le diamètre externe est de 25 mm.

Le profil des vis utilisées est le suivant :
- une zone de transport de 225 mm
- une zone de malaxage de 100 mm
- une seconde zone de transport de 575 mm

Les températures de chauffe affichées, allant de la zone d'alimentation à la filière s'élèvent à 55°C - 75°C - 80°C - 85°C - 85°C - 85°C - 90°C - 90°C - 90°C - 90°C - 90°C - 100°C.

La vitesse de rotation des vis est programmée 210 t/min.

Sur des éprouvettes du film ainsi produit, on mesure ensuite l'épaisseur du film, son allongement à la rupture et son module d'élasticité.

L'allongement à la rupture est mesuré par traction en milieu ambiant (23°C et humidité 50 %) sur des éprouvettes de 200 mm de longueur et 25 mm de largeur, la vitesse de traction étant de 200 mm/min.

Le module d'élasticité, exprimé en MPa, est déterminé par la force initiale d'allongement.

Les résultats obtenus sont les suivants :

| | |
|---|---|
| épaisseur | 0,256 mm |
| allongement | 457 % |
| module | 47 MPa |

### Exemple 2

On opère comme dans l'exemple 1, mais en remplaçant l'hexapropylméthylcellulose par une proportion identique d'hexapropylcellulose.

Les résultats obtenus sont les suivants :

| | |
|---|---|
| épaisseur | 0,311 mm |
| allongement | 515 % |
| module | 71 MPa |

### Exemple 3R

A titre d'exemple comparatif, on a réalisé un film, par pressage, à partir du copolymère de l'exemple 1 exempt de tout additif.

Les résultats obtenus sont les suivants :

| | |
|---|---|
| épaisseur | 0,110 mm |
| allongement | 2 % |
| module | 3562 MPa |

Il apparait que ce film présente un allongement à la rupture et un module d'élasticité qui ne conviennent nullement pour son utilisation sous forme de pansement médical.

### Exemples 4 à 12

En utilisant le mode opératoire de l'exemple 1, on a réalisé une série de films à partir de compositions reprises dans le tableau 1 ci-après et on a mesuré leur épaisseur et leur perméabilité à l'eau.

Les résultats obtenus sont repris dans le tableau 1

**TABLEAU 1**

| Formulation | perméabilité (g/m2.24h) | épaisseur mm |
|---|---|---|
| PLG* + 15 pcr TRIB* | 86 | 0,110 |
| PLG + 15 pcr TRIB + 10 HPMC* | > 9000 | 0,120 |
| PLG + 15 pcr TRIB + 15 HPMC | > 9000 | 0,100 |
| PLG + 20 pcr TRIB | 105 | 0,100 |
| PLG + 20 pcr TRIB + 10 HPMC | >9000 | 0,120 |
| PLG + 20 pcr TRIB + 15 HPMC | >9000 | 0,100 |
| PLG + 25 pcr TRIB | 129 | 0,090 |
| PLG + 25 pcr TRIB + 10 HPMC | >9000 | 0,090 |
| PLG + 25 pcr TRIB + 15 HPMC | >9000 | 0,100 |

| | | |
|---|---|---|
| *PLG = Copolymère de l'exemple 1 TRIB = Tributanoate de glycérol HPMC = Hexapropylméthylcellulose | | |

## Revendications

1. Film souple, élastique et biodégradable en polymère à base d'acide lactique pouvant notamment convenir pour la réalisation de pansements médicaux caractérisé en ce qu'il est produit par extrusion du polymère à base d'acide lactique préalablement plastifié par incorporation de 5 à 30 % en poids du polymère d'un ester de glycérol obtenu à partir d'un acide contenant 4 atomes de carbone dans sa molécule.

2. Film selon la revendication 1 caractérisé en ce qu'il présente un allongement à la rupture d'au moins 300 %.

3. Film selon la revendication 1 caractérisé en ce que le polymère à base d'acide lactique est un homopolymère de l'acide-L-lactique.

4. Film selon la revendication 1 caractérisé en ce que le polymère à base d'acide lactique est un copolymère d'acide-DL-lactique et d'acide glycolique contenant de 25 à 75 en poids d'unités dérivées d'acide lactique.

5. Film selon l'une quelconque des revendications 1 à 4 caractérisé en ce que le plastifiant est le tributanoate de glycérol.

6. Film selon l'une quelconque des revendications 1 à 5 caractérisé en ce que le polymère à base d'acide lactique extrudé contient, en outre, de 1 à 30 % en poids du polymère d'une cellulose alkylée.

7. Film selon la revendication 6 caractérisé en ce que la cellulose alkylée est choisie dans le groupe formé par l'hexapropylcellulose et l'hexapropylméthylcellulose.

8. Film selon l'une quelconque des revendications 1 à 7 caractérisé en ce qu'il contient, en outre, un produit pharmaceutique.

9. Pansement médical réalisé à partir d'un film obtenu suivant l'une quelconque des revendications 1 à 8.

## Claims

1. Flexible, elastic and biodegradable film made of polymer based on lactic acid, capable of being suitable especially for the production of medical dressings, characterised in that it is produced by extrusion of the lactic acid-based polymer plasticised beforehand by incorporation of 5 to 30 % by weight of the polymer of a glycerol ester obtained from an acid containing 4 carbon atoms in its molecule.

2. Film according to Claim 1, characterised in that it has an elongation at break of at least 300 %.

3. Film according to Claim 1, characterised in that the lactic acid-based polymer is a homopolymer of L-lactic acid.

4. Film according to Claim 1, characterised in that the lactic acid-based polymer is a copolymer of DL-lactic acid and of glycolic acid, containing from 25 to 75 by weight of units derived from lactic acid.

5. Film according to any one of Claims 1 to 4, characterised in that the plasticiser is glycerol tributanoate.

6. Film according to any one of Claims 1 to 5, characterised in that the lactic acid-based extruded polymer additionally contains from 1 to 30 % by weight of the polymer of an alkylated cellulose.

7. Film according to Claim 6, characterised in that the alkylated cellulose is chosen from the group consisting of hexapropyl cellulose and hexapropyl methyl cellulose.

8. Film according to any one of Claims 1 to 7, characterised in that it additionally contains a pharmaceutical product.

9. Medical dressing produced from a film obtained according to any one of Claims 1 to 8.

## Patentansprüche

1. Weicher elastischer und bioadsorbierbarer Film aus Milchsäurepolymer, der sich insbesondere für die Herstellung von medizinischen Verbänden eignet, dadurch gekennzeichnet, daß er durch Extrusion des Milchsäurepolymers hergestellt wird, das zuvor durch Einführen von 5 bis 30% des Polymergewichts eines aus einer Säure mit 4 Kohlenstoffatomen in ihrem Molekül erhaltenen Glyzerinesters weichgemacht wurde.

2. Film gemäß Anspruch 1, dadurch gekennzeichnet, daß er eine Reißdehnung von wenigstens 300% aufweist.

3. Film gemäß Anspruch 1, dadurch gekennzeichnet, daß das Milchsäurepolymer ein L-Milchsäurehomopolymer ist.

4. Film gemäß Anspruch 1, dadurch gekennzeichnet, daß das Milchsäurepolymer ein DL-Milchsäure-Glykolsäure-Copolymer ist, das 25 bis 75 Gew.-% von Milchsäure abgeleitete Einheiten enthält.

5. Film gemäß einem der Ansprüche 1 bis 4, dadurch gekennzeichnet, daß der Weichmacher Glyzerintributanoat ist.

6. Film gemäß einem der Ansprüche 1 bis 5, dadurch gekennzeichnet, daß das extrudierte Milchsäurepolymer außerdem 1 bis 30% des Polymergewichts einer Alkylzellulose enthält.

7. Film gemäß Anspruch 6, dadurch gekennzeichnet, daß die Alkylzellulose aus der aus Hexapropylzellulose und Hexapropylmethylzellulose gebildeten Gruppe ausgewählt ist.

8. Film gemäß einem der Ansprüche 1 bis 7, dadurch gekennzeichnet, daß er außerdem ein pharmazeutisches Produkt enthält.

9. Medizinischer Verband, der aus einem gemäß einem der Ansprüche 1 bis 8 erhaltenen Film hergestellt ist.
